# EUROPEAN PATENT APPLICATION

(11) **EP 4 840 482 A1**
(43) Date of publication of application: **18.11.2026**
(21) Application number: 24916117.5
(22) Date of filing: 12.01.2024
(51) Int. Cl.: A61M 1/00

(54) **DRAINAGE STRUCTURE FOR CONTINUOUS AND UNIFORM NEGATIVE-PRESSURE CONDUCTION ON ALL WOUND PROFILES**

(71) Applicant: Cheng, Hu, Wuhan, Hubei 430000 (CN)
(72) Inventor: Cheng, Hu, Wuhan, Hubei 430000 (CN)
(74) Representative: De Arpe Tejero, Manuel
(86) International application number: PCT/CN2024/072168
(87) International publication number: WO 2025/148053

(57) **Abstract**

The present disclosure relates to a drainage structure for continuous and uniform negative pressure conduction of a full wound surface, used in conjunction with a porous foam layer for negative pressure drainage operation, comprising a structural body, and partitions are disposed on one surface of the structural body that faces the porous foam layer , the partitions forms cavities between the structural body and the porous foam layer, and through holes are provided on the structural body at positions corresponding to the cavities, allowing the spaces on opposite sides of the structural body to be connected. The drainage structure employs a single-layer structure to enhance the negative pressure conduction effect. In clinical applications, When it used on uneven wound surfaces or cavity gaps, does not produce interlayer displacement with the negative pressure,. When it is used together with PVA porous foam material, it provides a path that does not dry out and collapse, and also offers some moisture retention. Therefore, it solves the problems that PVA porous foam tends to face as it loses water, like gradually hardening, decreased resilience and permeability of the foam, and the eventual blockage of the drainage channels.

## Description

### TECHNICAL FIELD

The present disclosure relates to the technical field of vacuum sealing drainage (VSD), and specifically refers to a drainage structure for continuous and uniform negative pressure conduction of a full wound surface.

### BACKGROUND

Existing VSD technologies include a medical hydrophilic foam material or a medical hydrophobic foam material (collectively referred to as a porous foam material), a sealing film, drainage pipes, a collection container and a negative pressure source (including a medical suction machine, a hospital central negative pressure device or a negative pressure drainage bottle). By applying a continuous or intermittent negative pressure suction to the wound surface or wound cavity, harmful substances such as exudate, necrotic tissues, toxins and pus in the wound can be drained into the collection container, thereby directly discharging the exudate, the necrotic tissue, the toxins and the pus, thereby avoiding the reabsorption of harmful substances by liver and kidney, reducing the amount of bacteria on the wound surface, reducing edema, and preventing the occurrence of multiple organ failure (MOF) and acute respiratory distress syndrome (ARDS). At the same time, the negative pressure suction can increase the blood flow rate of the wound, stimulate the cells to secrete growth factors, thereby effectively promoting growth of neovascularization and granulation tissue, promoting tissue repair, and shortening the healing time of wound surface and wound cavity. By using the technology, cross infection is effectively avoided, the workload of medical staff is reduced, the cure rate of the wound is improved, the mortality and the disability of the wound are reduced.

Currently, the VSD technology achieves a complete therapeutic system of coordinated symbiosis by performing "Sealing" + "Vacuum" + "Drainage" three consecutive actions; the process of coordinated symbiosis is as follows:

The sealing of wound is completed by a foam material, and the sealing is the most basic necessary medical procedure for transforming the open wound surface into a closed wound surface in clinical surgery; at the same time, after the wound surface is closed, the wound area changed from a plane shape to a capsule shape, and the capsule shape provides a space for the next step of negative pressure to take effect.

Vacuum is performed by an externally connected drainage pipes and a negative pressure source; The negative pressure here refers to the pressure in the capsule space that is much lower than the blood pressure in the capillary vessel of the wound tissue after a negative pressure source performs work, thus creating negative pressure. Since the pressure in the capillary vessel is much greater than the pressure outside the capillary vessel, the negative pressure effect on the wound surface will directly affect the dilation and bulging of the capillary wall in the wound; Due to the expansion and bulge, the space volume of the capillary volume is increased, and the increase of the volume has two effects: first, the increase of the blood flow rate and the blood flow velocity is 5-12 times or more, which brings a large number of tissue progenitor cells necessary for wound repair, oxygen-rich red blood cells, and platelet-rich plasma (PRP) rich in repair growth factors and various amino acid components for generating tissue proteins. Second, the local volume of wound surface increase continuously, which will cause the network-like capillary vessel to expand, twist and clump toward the sidewall of the blood vessel, thereby forming a neatly arranged granulation tissue bed, such as rubies.

Drainage refers to the connection between a negative pressure source and the capsule space of the wound through a drainage pipe; Drainage brings: 1) a negative pressure source is provided for the capsule space of the wound, causing the pressure in the capillary vessel in the wound tissue is much greater than the pressure in the capsule space. As a result, the blood flow rate and blood flow velocity in the capillary vessel are increased by 5 to 12 times than other tissues outside the wound; 2) contaminants, necrotic tissue and necrotic tissue debris, pus, tissue exudate, etc. on the wound surface are continuously and quickly removed to maintain cleanliness.

Thus, Vacuum, Sealing, Drainage forms a mutually reinforcing and efficient treatment system, i.e. VSD.

However, current clinical application of VSD still has significant limitations, including at least the following:
(1) The porous foam material is prone to drying and hardening due to loss of water after 2-4 days of clinical application, which causes the material to lose its resilience, the effective drainage cavity gap is greatly reduced, permeability decreases, blockage gradually occurs, and the pores of the foam material are often adhered under the action of organic colloids such as pus, interstitial fluid, exudate, blood, etc. and the foam material covered on the wound is actually inelastic.
(2) when the drainage device with a double-layer structure is applied to the surface of an uneven wound surface, under the condition of negative pressure, in the bending state, the curvature generated by the deformation of the upper and lower structures is different, so that the shear force on the body surface endothelial cells will be generated on the surface of the concave uneven surface, thereby damaging the tissue of the endothelial cell granulation tissue, so that the wound tissue bed loses the repair activity.
(3) The bottom of the double-layer drainage device sticks to a PVA layer, but because the curvature changes between layers, it's easy for the PVA layer to peel off, causing multiple layers to shift. This makes the entire negative-pressure drainage system on the wound loose, and it can even leak or collapse.

### SUMMARY

In view of the shortcomings of the prior art, the present disclosure provides a drainage structure for continuous and uniform negative pressure conduction of a full wound surface, that uses a single-layer design, enhancing negative pressure transmission and wound drainage, greatly reducing resistance to negative pressure transmission, and evenly distributing the negative pressure across the entire wound. Meanwhile, the hydrophilic PVA material provides ongoing wound hydration within a flexible space, and the simple single-layer support and drainage structure also avoids deformation and displacement seen in other multi-layer designs.

In order to achieve the above objective, the drainage structure, which is used in conjunction with a porous foam layer for negative pressure drainage operation, comprising a structural body, and partitions are disposed on one surface of the structural body that faces the porous foam layer, the partitions forms cavities between the structural body and the porous foam layer , and through holes are provided on the structural body at positions corresponding to the cavities, allowing the spaces on opposite sides of the structural body to be connected.

Preferably, each of the partitions comprises a fixed end and a top end, the fixed end is fixed to the structural body; the volume of the partitions is tapered from the fixed end to the top end, so that the inner surface of the cavities is gradually expanded from the fixed end to the top end. Under the negative pressure condition, the gradually expanding surface gradually increases the contact area between the porous foam layer and the partitions, thereby gradually reducing the pressure on the partitions. As a result, the pressure transmitted by the partitions to the wound surface decreases gradually and uniformly, thus lowering the pressure on the surface of the granulation tissue to the minimum physiological level.

Preferably, the fixed end has a round or a regular polygon end face, and wherein the top end terminates in a point or a plane. The round or regular polygon end face is connected to the surface of the structural body, and the top end is connected to the porous foam layer.

Preferably, a ratio of the distance d2 of the adjacent partitions on the structural body to a bottom width d1 of one of the partitions is in a range of 0.2 to 3. If the distance d2 between adjacent partitions is too large, the partitions can't provide the rebound effect. the porous foam layer and the structural body may not be separated due to adhesion of the residual colloidal substance in the drainage fluid; If the spacing is too small, which easily causes the accumulation of various colloidal-like drainage substances, so that the drainage substances cannot be discharged out of the wound in a timely manner.

Preferably, the partitions are rotating bodies, and the generatrix of the rotary body is a straight generatrix or a curved generatrix. This kind of rotating body design makes the partitions' surface smooth and spreads the force evenly.

Preferably, a shape of the axial section of the partitions is selected from one or two of the following: triangle, trapezoid, circle, semicircle, ellipse, rectangle, and parallelogram.

Preferably, the partitions are columns, with multiple flanges extending outward along an axial direction thereof.

Preferably, each of the flanges comprises an arc end or a conical end.

Preferably, the drainage structure further comprises multiple protrusions disposed on the partitions, wherein the protrusions are selected from the group consisting of: (i) evenly spaced protrusions, and (ii) unevenly spaced protrusions. The protrusions are used to better bond with PVA material in the flocking process.

Preferably, the structural body is connected to the porous foam layer on one surface close to a wound, and the other surface away from the wound is connected to one or more suction cups.

Preferably, the porous foam layer is made of one or more of the following materials: polyvinyl alcohol resin, modified polyvinyl alcohol resin, polyurethane elastomer, and modified polyurethane elastomer.

Preferably, the drainage structure further comprises a sealing film, which is attached to the other surface of the structural body that faces away from the wound, and the sealed film has drainage holes, the skirts of the suction cups are attached to the semipermeable membrane, and the central cavity of each of the suction cups is aligned with one of the drainage holes.

Preferably, the other surface of the structural body that faces away from the wound is a rough planar surface, used to provide damping for the sealing film and prevent it from shifting.

Preferably, the structural body and the porous foam layer are fixedly connected by sewing or gluing, so the structural body and the porous foam layer can be cut and spliced according to the size of the wound.

Preferably, the fixing points between the structural body and the porous foam layer are uniformly distributed to ensure effective fixation of the cutting area after cutting.

Preferably, the structural body is flocked onto a surface of the porous foam layer via a coating and heat-drying process.

Preferably, the partitions are evenly spaced on one surface of the structural body that faces the wound.

Preferably, the diameter of the bottom of each of the partitions is not greater than 10 mm, and the height of each of the partitions is not greater than 6 mm.

Preferably, the porosity of the structural body is 15% -70%.

Preferably, the materials of the structural body and the partitions are made of thermosetting elastomers, rubber, or thermoplastic elastomers.

Preferably, the structural body is formed by a replica molding process, an injection molding process, or a compression molding process.

Preferably, the hardness range of the structural body is 5-35 Shore A, and the hardness range of the partitions is 5-40 Shore A. The hardness of the partitions may be slightly higher than the structural body to give the structural body an appropriate resilience.

In the negative pressure drainage therapy, the drainage structure of the present disclosure is used together with a porous foam layer. The partitions on the underside of the structural body creates cavities between the structural body and the porous foam layer, forming an effective conduction channel on the surface of the porous foam layer. This also reduces the pressure on the porous foam layer against the wound, significantly lessening the compression on the capillary endothelial cells in the granulation tissue bed. After drainage, the partitions help the structural body bounce back upward, gradually restoring the cavities volume, which maintains a relatively stable space between the structural body and the porous foam layer, effectively restoring the drainage gap.

Compared with the prior art, the drainage structure of the present disclosure has the following effects:
1. The design of the partitions ensures that the support columns 'will not tip, tilt, or shift during negative pressure suction, making it suitable for use on any wound surface, including twisted, stepped, spiraled, or even folded shapes.
2. In actual use, the interval changes between the maximum negative pressure value and the minimum value required for clinical wound treatment are relatively large, and the gradual gentle deformation of the cambered surface curvature of the partitions provides a space that can adapt to the pressure change drastically while ensuring continuous effective drainage and continuous rebound for the wound negative pressure drainage treatment.
3. The spacing of the partitions is designed to be within a reasonable range, so as to prevent the porous foam layer and the structural body from being adhered without separation due to excessive spacing; and if the spacing is too small, it will easily cause the accumulation of various colloidal-like drainage substances, so that the drainage substances cannot be discharged promptly from the wound.
4. In the negative pressure drainage operation, as the pressure keeps increasing, the pressure on the wound also keeps going up. During this process, the contact area between the partitions and the wound gradually increases. Compared to using straight-column partitions, the pressure on the wound is significantly reduced, which greatly reduces the likehood of blocking blood flow in the wound tissue or causing compressive necrosis of the tissue cells.
5. When used clinically on uneven wound surfaces or cavity spaces, the drainage structure will not cause layers to shift or shear under negative pressure.
6. When the drainage structure is used together with PVA porous foam material, it provides a path that does not dry out and collapse, and also offers some moisture retention. This helps solve the problems that PVA porous foam tends to face as it loses water, like gradually hardening, decreased resilience and permeability of the foam, and the eventual blockage of the drainage channels.
7. In clinical practice, using this drainage structure means fewer replacements, less chance of the wound being exposed to air, longer use of negative pressure drainage materials in the wound cavity, less work for medical staff, less pain for patients when changing dressings, significantly lower medical costs, and greatly improved treatment efficiency.

### BRIEF DESCRIPTION OF THE DRAWINGS

FIG. 1 is a schematic front view of Embodiment 1.
FIG. 2 is a schematic bottom view of Embodiment 1.
FIG. 3 is a schematic top view of Embodiment 1.
FIG. 4 is a schematic front view of a single partition with an axial section of a trapezoid in Embodiment 2.
FIG. 5 is a three-dimensional structural schematic diagram of a single partition with an axial section being triangular in Embodiment 2.
FIG. 6 is a schematic front view of a single partition with an axial section of a truncated cone in Embodiment 2.
FIG. 7 is a three-dimensional structural schematic diagram of a single partition with an axial section of a truncated cone in Embodiment 2.
FIG. 8 is a schematic front structural view of a single partition with an elliptical axial cross-section in Embodiment 2.
FIG. 9 is a three-dimensional structural schematic diagram of a single partition with an elliptical axial cross-section in Embodiment 2.
FIG. 10 is a schematic front view of a single partition with an outer flange in Embodiment 3.
FIG. 11 is a three-dimensional structural schematic diagram of a single partition with an outer flange structure in Embodiment 3.
FIG. 12 is a schematic front structural view of a drainage structure in Embodiment 4.
FIG. 13 is a schematic front view of a drainage structure in Embodiment 5.
FIG. 14 is a schematic front structural view of a drainage structure in Embodiment 6.
FIG. 15 is a schematic structural diagram of a drainage structure in Embodiment 6.
FIG. 16 is a schematic front view of a drainage structure in Embodiment 7.

In the figures: structural body 1, porous foam layer 2, partitions 3, fixed end 3-1, top end 3-2, cavities 4, through holes 5, sealing film 6, suction cups 7, and drainage hole 8.

### DETAILED DESCRIPTION OF THE EMBODIMENTS

The present disclosure will be further described in detail below with reference to the accompanying drawings and specific embodiments.

### Example 1

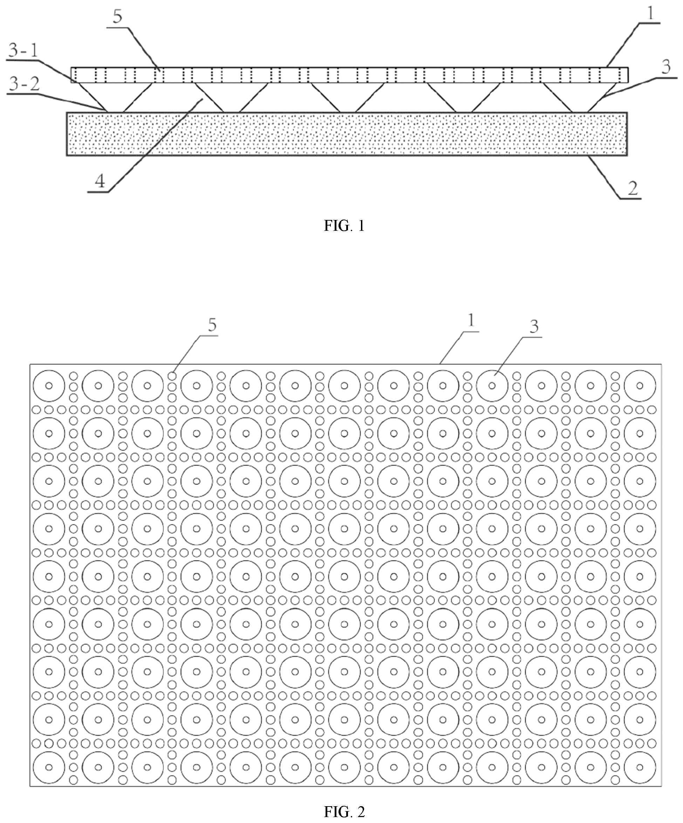

As shown in FIG. 1 to FIG. 3, a drainage structure for continuous and uniform negative pressure conduction of a full wound surface provided in this embodiment includes a structural body (1), and partitions (3) are disposed on one surface of the structural body (1) that faces the porous foam layer (2), the partitions (3) form cavities (4) between the structural body (1) and the porous foam layer (2), and through holes (5) are provided on the structural body (1) at positions corresponding to the cavities (4), allowing the spaces on opposite sides of the structural body (1) to be connected.

The structural body 1 and the porous foam layer 2 are of a uniform flexible structure. The structural body 1 may be a sheet structure, or roughly a layered structure, and on this basis, it has irregular shapes with indentations, protrusions, twists, and folds.

In the negative pressure drainage process, firstly, sealing is performed by means of the medical sealing film to complete sealing, the surface of the wound is artificially turned into a capsule shape after the wound surface is closed, and the capsule space is provided with a space for work under negative pressure; then, the external negative pressure device gives negative pressure to the capsule space. under the action of a powerful negative pressure in the capsule space, the pressure in the capillary vessel is much greater than the pressure outside the capillary vessel, and the negative pressure effect on the wound surface will directly affect the expansion and protrusion of the capillary wall in the wound surface; Under the action of negative pressure, the structural body 1 is compressed and deformed toward the negative pressure source, while the porous foam layer 2 below exerts an upward compressive force on the partitions 3, reducing the pressure between the porous foam layer 2 and the contact surface, and significantly alleviating the compression of capillary endothelial cells on the granulation tissue bed. And the partitions 3 are stressed simultaneously from the bottom surface and the sides to prevent the partitions 3 from being distorted and deformed; Next, a drainage tube is connected to a negative pressure source and the capsule space on the wound surface to turn on drainage. The drainage fluid is guided from the porous foam layer 2 to the cavities 4 and discharged through the through holes 5, and as the negative pressure increases, the volume of the cavities 4 is continuously compressed, and the contact area between the porous foam layer 2 and the partitions 3 gradually increases, so that the pressure received by the partitions 3 is reduced, thereby reducing the compression force of the wound. After the drainage is completed, the pressure in the wound cavity decreases, the porous foam layer 2 is no longer subjected to negative pressure, starts to rebound, and gradually moves away from the top end of the partitions 3, so that the volume of the cavities 4 are gradually restored, and the partitions 3 maintain the relative space between the structural body 1 and the porous foam layer 2, so that the effective drainage gap is restored.

### Example 2

The shape, structure, and arrangement of partitions 3 can have various designs and can be adaptively adjusted according to actual requirements.

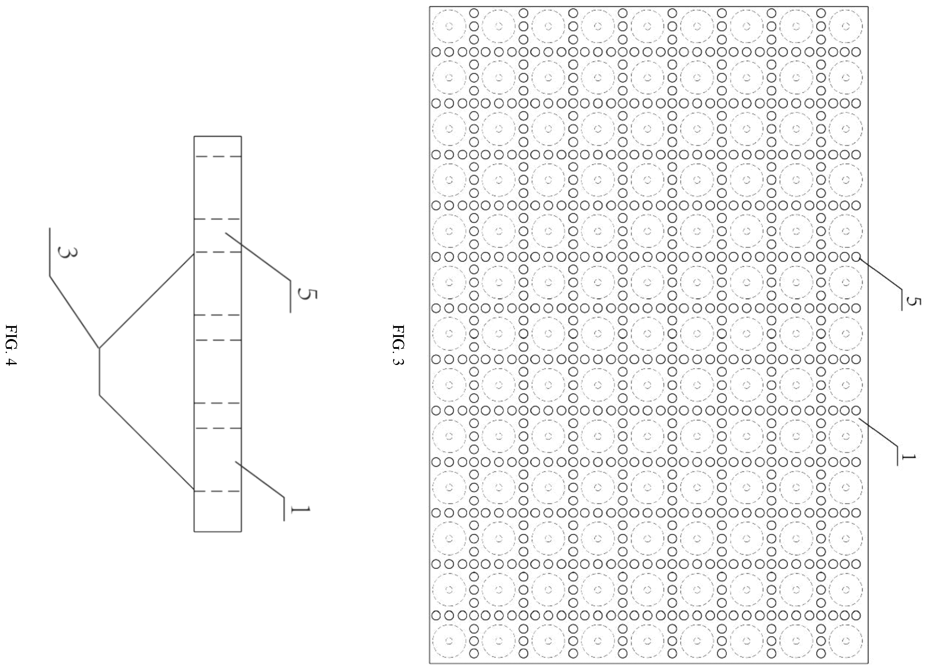

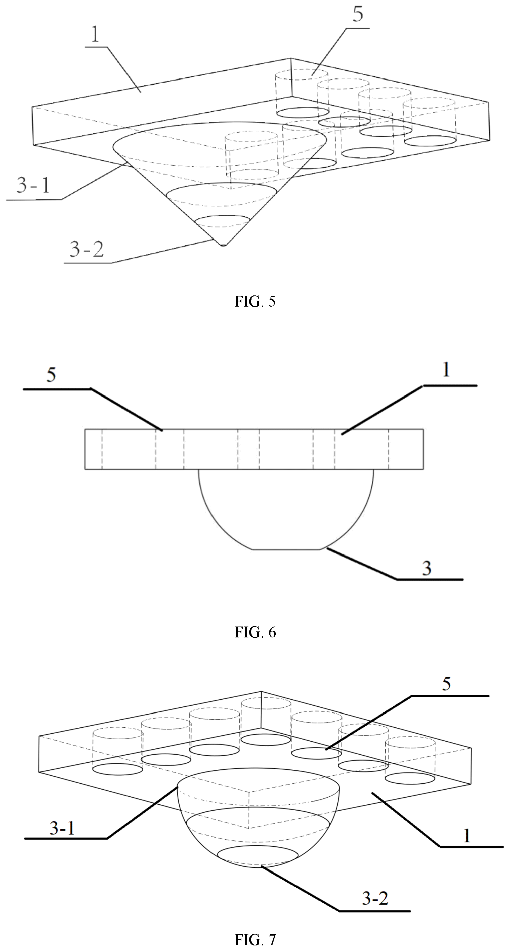

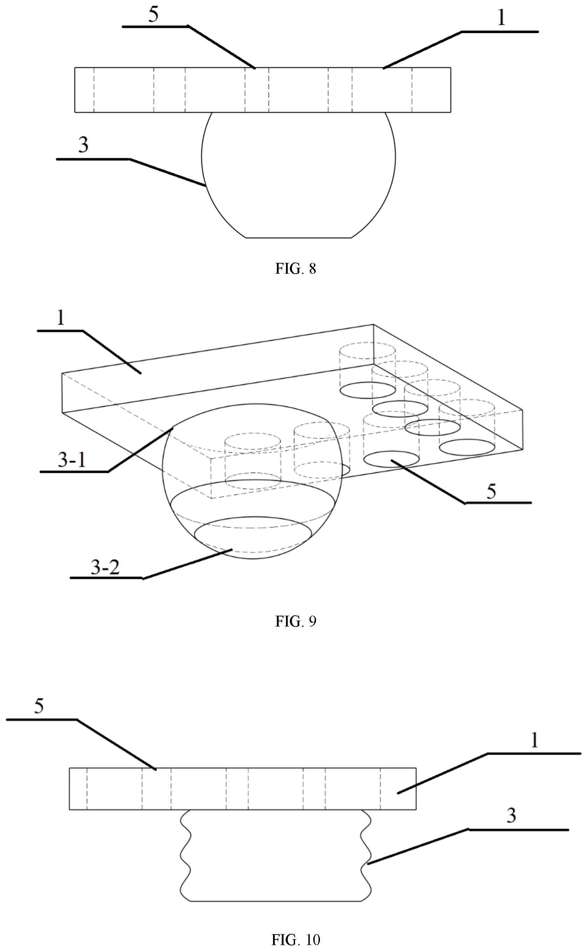

As an embodiment of the present disclosure, the partitions (3) are rotating bodies, and the generatrix of the rotary body is a straight generatrix. The shape of the axial section of the partitions (3) is selected from one or two of the following: triangle, trapezoid, circle, semicircle, frustum, ellipse, rectangle, and parallelogram. The axial section of the partitions 3 has a trapezoidal structure as shown in FIG. 4, the axial section is a triangular structure as shown in FIG. 5, the axial section is a frustum-shaped structure as shown in FIG. 6 and FIG. 7, and the axial section is an elliptical structure as shown in FIG. 8 and FIG. 9.
each of the partitions (3) comprises a fixed end (3-1) and a top end (3-2), the fixed end (3-1) is fixed to the structural body (1); the volume of the partitions (3) is tapered from the fixed end (3-1) to the top end (3-2), so that the inner surface of the cavities (4) is gradually expanded from the fixed end (3-1) to the top end (3-2).
the materials of the structural body (1) and the partitions (3) are made of thermosetting elastomers, rubber, or thermoplastic elastomers. The drainage structure may be formed by a replica molding process, an injection molding process, or a compression molding process. The effective porosity of the structural body 1 is preferably 15% -70%; the diameter of the bottom surface of the partitions 3 is not greater than 10 mm, and the height is not greater than 6 mm. The hardness range of the structural body 1 is 5-35 Shore A, and the hardness range of the partitions 3 is 5-40 Shore A.

During use, the structural body 1 is placed on the porous foam layer 2, or the two are fixedly connected through stitching or adhesive bonding. The structural body 1 is a sheet-like structure, and its shape can be cut and pieced together according to the shape and size of the wound or wound cavity. The fixation points between the structural body 1 and the porous foam layer 2 are evenly distributed, ensuring effective fixation within the cut area during trimming and using. The top end 3-2 of the partitions 3 contacts the porous foam layer 2, so that the downward protruding arc surface of the lower surface of the partitions 3 forms upwardly tapered cavities 4 on the upper surface of the porous foam layer 2

Under the action of negative pressure, the drainage fluid is conducted from the porous foam layer 2 to the cavities 4 and discharged through the through holes 5, since the end face of the top end 3-2 of the partitions 3 is smaller than the end face of the bottom surface of the fixed end 3-1, under the action of high negative pressure, the porous foam layer 2 will come into contact with the side surface of the partitions 3 after extrusion deformation, and the arc-shaped side surface increases the contact area of the porous foam layer 2 and the partitions 3, so that the pressure received by the partitions 3 is reduced, thereby reducing the compression force of the wound surface.

### Example 3

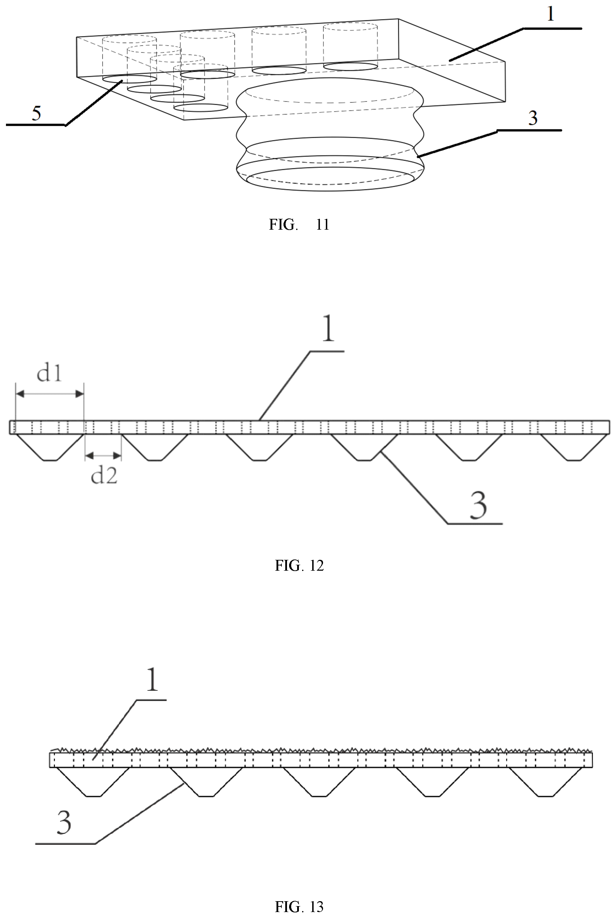

The drainage structure in this embodiment differs from Embodiment 1 in that the partition 3 is a rotary body of the columnar structure, but the generatrix of the rotary body is a curved generatrix, and multiple flanges extend outward along the axial direction of the partitions 3, as shown in FIG. 10 and FIG. 11, each of the flanges has an arc end or a conical end.

In this embodiment, when the partitions 3 is used, the flange structure increases the outer surface area of the partitions 3, and in the negative pressure drainage operation, the pressure received by the partitions 3 can be further reduced due to the larger outer surface area, so that the compression force of the drainage structure on the wound surface is small, and the pain of the patient is reduced.

Further, on the partition 3, the flanges with a toothed arrangement better maintains the cavities 4 between the structural body 1 and the porous foam layer 2, so that the structural body 1 and the porous foam layer 2 rebound immediately after the negative pressure drainage ends, and the partitions 3 made of the elastic material can provide a higher elastic coefficient, at the same time, the elastic partitions 3 can provide a high elastic coefficient, continuously offering rebound force like a spring during rebound, maintaining the effect of negative pressure transmission.

### Example 4

The drainage structure in this embodiment differs from Embodiment 1 in that, as shown in FIG. 12, the ratio of the distance d2 of the adjacent partitions (3) on the structural body (1) to a bottom width d1 of one of the partitions (3) is in a range of 0.2 to 3.

In this embodiment, 30 samples with a relatively large d2/d1 ratio (d2/d1 = 0.2-3) and 30 samples with a relatively small d2/d1 ratio were selected for performance testing. Under a negative pressure of 85 mmHg, continuous suction was applied for 7 days without irrigation. The test results are shown in Table 1:

**TABLE 1. Comparison of Experimental Effects of Ratio Change of D2/D1**

| d2/d1 ratio | Moisturizing performance | Drainage effect | Anti-clogging performance |
|---|---|---|---|
| 4 | After continuously sucking for 2 days, it becomes locally dry and hard. | The drainage time ranges from 30 to 120 seconds (when not completely hardened or only partially hardened), and after most areas have hardened, situations where drainage is impossible may occur. | 70% blocked on the 3rd day, completely blocked on the 7th day. |
| 1 | After continuously sucking for 3-5 days, it is still moist and soft. | The drainage time is 3-20 seconds | On the third day, there was nearly no obstruction, and on the 7th day, 90% of the passages were unobstructed. |
| 0.15 | After continuously sucking for 2 days, most areas become dry and hard. | Drainage time is between 3 and 120 seconds (if not fully solidified or only partially solidified), and in most areas, drainage may become impossible once it has fully solidified. | 80% blocked on the 3rd day, completely blocked on the 7th day |

The experimental results confirm that when the ratio of d2/d1 is large, the cavities 4 formed by the gradually expanding surface of the adjacent partitions 3 is too large, and in the case that the negative pressure is increased, the cavities 4 between the porous foam layer 2 and the structural body 1 that lacks the supporting of the partitions 3 gradually disappears, the porous foam layer 2 is in direct contact with the structural body 1. In this situation, the negative pressure applies compression to the wound without being attenuated by the partitions 3. After the drainage is completed, due to the lack of rebound force generated by the partitions 3, the porous foam layer 2 and the structural body 1 may not be separated due to adhesion of the residual colloidal substance in the drainage fluid, which causes the volume of the cavities 4 to be reduced, so that the structural body 1 starts locally drying and hardening from the upper surface, and gradually causes large-area dry hard and comprehensive blockage.

When the ratio of d2/d1 is within a reasonable range of 0.2 to 3, after continuous suction for 3-5 days, structural body 1 is still able to remain moist and soft; the drainage efficiency is not hindered, there is no blockage, and the drainage effect is good.

When the ratio of d 2/d 1 is small, the cavities 4 formed by the gradually expanding surface of the adjacent partitions 3 is small, which easily causes drainage fluid accumulation and cannot be discharged in a timely manner through the through holes 5, and the gelatinous substance in the drainage fluid easily causes the through-hole blockage to occur.

### Example 5

The drainage structure in this embodiment differs from Embodiment 1 in that the upper surface of the structural body 1 is a non-smooth rough plane, as shown in FIG. 13.

The upper surface of the structural body 1 is sealed by the sealing film 6, and a frictional resistance is formed between the structural body 1 having the rough surface and the sealing film 6 to prevent displacement of the structural body 1 relative to the sealing film 6, thereby avoiding the risk of loosening or detachment.

### Example 6

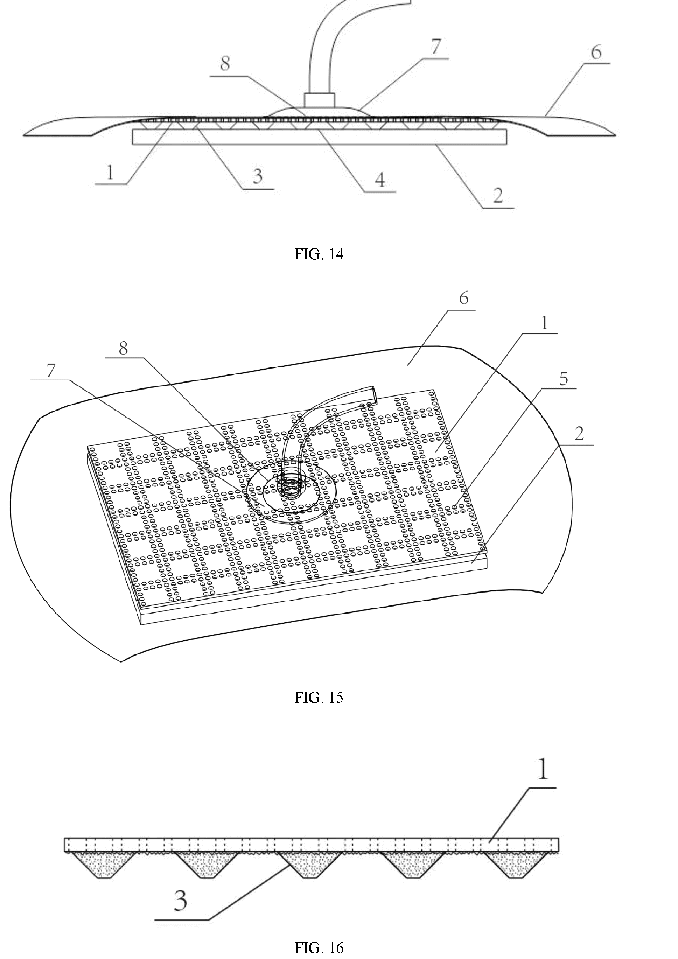

The drainage structure in this embodiment, the structural body (1) is connected to the porous foam layer (2) on one surface close to a wound, and the other surface away from the wound is connected to one or more suction cups (7), to facilitate sealing with the sealing film 6, as shown in FIG. 14. The sealing film 6 is provided with drainage holes 8, the skirts of the suction cups (7) are attached to the semipermeable membrane, and the central cavity of each of the suction cups (7) is aligned with one of the drainage holes (8), as shown in FIG. 15.

After sealing, the drainage structure can be detachably connected to the external negative pressure device and the drainage bag through the suction cup 7, and after the connection, the external negative pressure device is started, that is, the whole system starts to perform negative pressure suction.

### Example 7

As shown in FIG. 16, the present embodiment provides another preferred structure of drainage structure, which differs from Embodiment 1 in that the outer surface of the partition 3 is provided with protrusions distributed in a dense regular or irregular distribution, and the convex front section can be designed as a sharp, conical, or chamfered protrusion.

Using a coating process, PVA is flocked onto the lower surface of structural body 1 after thermal baking. During this process, the raw foam material is heated, causing the gas mixed within to expand, forming rising bubbles as the liquid evaporates. As these bubbles ascend, they create channels and cavities. The small bubbles in the topmost layer gradually rupture, and when the ruptured small bubbles encounter the protrusions on the outer surface of partitions 3, they solidify at the gaps between the protrusions and the bubbles, adhering firmly to the outer surface of partitions 3.

According to the above seven structures provided by the present disclosure, when the negative pressure suction is performed, under the action of negative pressure, the structural body forms conductive cavities around the partitions; In actual clinical use, in tissues close to the heart like the abdomen, back, and well-supplied facial areas, applying a negative pressure of 120mmHg keeps the partitions 3 stable and firm without tipping over, thus forming multiple stable conductive cavities. For a poor blood supply distal end, when the suction negative pressure is up to 400 mmHg, the conductive cavities are still stable. When the lower layer of small pores is blocked by a blood clot or a purulent gel-like substance, the plurality of conductive cavities can still conduct negative pressure and suck the blood clot or the gel-like substance, thereby solving the problem of plugging.

The present disclosure greatly reduces the compression area of the wound on the wound, and is superior to the PVA material, the PU material and the gauze, the reason is that: 1. the partitions structure forms an LCP (limited contact plane limiting point contact surface), and a bridge-like connecting net is formed on the wound surface and the granulation tissue bed to provide sufficient space for the growth of the granulation; 2. The LCP formed by the partitions of the present disclosure is far less than formed by PVA material, PU material or gauze, so that the compression of the wound material on the granulation is more effectively reduced; and 3. the pressure of the LCP unit area of the partitions structure of the present disclosure is much lower than the pressure of the LCP of the PVA material, the PU material and the gauze.

The drainage structure of the present disclosure can be used for the wound surface and can also be used in an in vivo wound cavity, and when used for surface wound surface wound, the lower surface of the drainage structure is designed as a PVA flocking structure or the use of the chimeric PVA wound material, which makes the material more affinity with wound tissue, and also provides an optimal wet healing environment for healthy growth of endothelial cells of the meat bud tissue; When used in an in-vivo wound cavity, since the inner wall of the deep cavity is always wet, and the drainage structure is soft and easy to bend, the partitions structure avoids the compression of the granulation tissue to the greatest extent, so that the wound treatment effect will be significantly improved compared with the use of the PVA material, the PU material, the gauze material, etc. simply by using the drainage structure of the present disclosure.

The drainage structure of the present disclosure has excellent therapeutic effects on surface wounds with complex wound terrain, and is suitable for perineal, anus, axilla, submandibular burns, explosive injuries, lacerations, and crush-explosive injuries of the limbs. Because of the gradual reduction of the arc curvature of the partitions, the absolute support height reaches the relatively optimal value required for wound healing physiology. The number, height and curvature of the partitions determine the number and size of the conductive cavities, and further determine the anti-blocking effect; in order to ensure the stability of the partitions, the diameter of the bottom surface of the partitions is set to not be greater than 10 mm, the height is set to not be greater than 6 mm, and the hardness is set to 5-40 Shore A.

The drainage structure of the present disclosure, due to its porous and multi-support design, ensures smooth drainage without blockage; when used in combination with PVA material, it provides a passage that does not harden or collapse, and also has a certain moisturizing effect, making the PVA material almost never dry out or harden, while significantly improving its resilience and permeability; it is easy to seal, easy to rinse, and allows for effortless drainage, thereby significantly enhancing treatment efficacy and efficiency.

The specific embodiments of the present disclosure have been described in detail above, but are merely examples, and the present disclosure is not limited to the specific embodiments described above. Any equivalent modifications and alternatives to the present disclosure will be within the scope of the present disclosure to those skilled in the art. Therefore, equivalent transformations and modifications made without departing from the spirit and scope of the present disclosure shall fall within the scope of the present disclosure.

## Claims

1. A drainage structure for continuous and uniform negative pressure conduction of full wound surface, which is used in conjunction with a porous foam layer (2) for negative pressure drainage operation, comprising: a structural body (1), and partitions (3) are disposed on one surface of the structural body (1) that faces the porous foam layer (2), the partitions (3) form cavities (4) between the structural body (1) and the porous foam layer (2), and through holes (5) are provided on the structural body (1) at positions corresponding to the cavities (4), allowing the spaces on opposite sides of the structural body (1) to be connected.

2. The drainage structure according to claim 1, wherein each of the partitions (3) comprises a fixed end (3-1) and a top end (3-2), the fixed end (3-1) is fixed to the structural body (1); the volume of the partitions (3) is tapered from the fixed end (3-1) to the top end (3-2), so that the inner surface of the cavities (4) is gradually expanded from the fixed end (3-1) to the top end (3-2).

3. The drainage structure according to claim 2, wherein the fixed end (3-1) has a round or a regular polygon end face, and wherein the top end (3-2) terminates in a point or a plane.

4. The drainage structure according to claim 1, wherein a ratio of the distance d2 of the adjacent partitions (3) on the structural body (1) to a bottom width d1 of one of the partitions (3) is in a range of 0.2 to 3.

5. The drainage structure according to claim 1, wherein the partitions (3) are rotating bodies, and the generatrix of the rotary body is a straight generatrix or a curved generatrix.

6. The drainage structure according to claim 1, wherein a shape of the axial section of the partitions (3) is selected from one or two of the following: triangle, trapezoid, circle, semicircle, ellipse, rectangle, and parallelogram.

7. The drainage structure according to claim 1, wherein the partitions (3) are columns, with multiple flanges extending outward along an axial direction thereof.

8. The drainage structure according to claim 7, wherein each of the flanges comprises an arc end or a conical end.

9. The drainage structure according to claim 1, further comprising multiple protrusions disposed on the partitions (3), wherein the protrusions are selected from the group consisting of: (i) evenly spaced protrusions, and (ii) unevenly spaced protrusions.

10. The drainage structure according to claim 1, wherein the structural body (1) is connected to the porous foam layer (2) on one surface close to a wound, and the other surface away from the wound is connected to one or more suction cups (7).

11. The drainage structure according to claim 10, wherein the porous foam layer (2) is made of one or more of the following materials: polyvinyl alcohol resin, modified polyvinyl alcohol resin, polyurethane elastomer, and modified polyurethane elastomer.

12. The drainage structure according to claim 10, further comprising a sealing film (6), which is attached to the other surface of the structural body (1) that faces away from the wound, and the sealed film (6) has drainage holes (8), the skirts of the suction cups (7) are attached to the semipermeable membrane, and the central cavity of each of the suction cups (7) is aligned with one of the drainage holes (8).

13. The drainage structure according to claim 12, wherein the other surface of the structural body (1) that faces away from the wound is a rough planar surface.

14. The drainage structure according to claim 1, wherein the structural body (1) and the porous foam layer (2) are fixedly connected by sewing or gluing.

15. The drainage structure according to claim 14, wherein the fixed points between the structural body (1) and the porous foam layer (2) are uniformly distributed.

16. The drainage structure according to claim 1, wherein the structural body (1) is flocked onto a surface of the porous foam layer (2) via a coating and heat-drying process.

17. The drainage structure according to any one of claims 1 to 16, wherein the partitions (3) are evenly spaced on one surface of the structural body (1) that faces the wound.

18. The drainage structure according to any one of claims 1 to 16, wherein the diameter of the bottom of each of the partitions (3) is not greater than 10 mm, and the height of each of the partitions (3) is not greater than 6 mm.

19. The drainage structure according to any one of claims 1 to 16, wherein a porosity of the structural body (1) is 15% -70%.

20. The drainage structure according to any one of claims 1 to 16, wherein the materials of the structural body (1) and the partitions (3) are made of thermosetting elastomers, rubber, or thermoplastic elastomers.

21. The drainage structure according to any one of claims 1 to 16, wherein the structural body (1) is formed by a replica molding process, an injection molding process, or a compression molding process.

22. The drainage structure according to any one of claims 1 to 16, wherein a hardness range of the structural body (1) is 5-35 Shore A, and a hardness range of the partitions (3) is 5-40 Shore A.
